(19) Europäisches Patentamt
European Patent Office
Office européen des brevets

(11) Veröffentlichungsnummer: **0 167 875**
**B1**

(12) # EUROPÄISCHE PATENTSCHRIFT

(45) Veröffentlichungstag der Patentschrift: **01.06.88**

(51) Int. Cl.⁴: **C 07 C 93/06**, C 07 C 89/00

(21) Anmeldenummer: **85107327.0**

(22) Anmeldetag: **13.06.85**

(54) **Verfahren zur Herstellung von reinen 2-Ethylamino-4-nitro-1-alkoxybenzolen.**

(30) Priorität: **20.06.84 DE 3422807**

(43) Veröffentlichungstag der Anmeldung: **15.01.86 Patentblatt 86/3**

(45) Bekanntmachung des Hinweises auf die Patenterteilung: **01.06.88 Patentblatt 88/22**

(84) Benannte Vertragsstaaten: **CH DE FR GB IT LI**

(56) Entgegenhaltungen:
**EP - A - 0 019 158**
**DE - A - 2 737 137**

**TETRAHEDRON LETTERS, Band 25, Nr. 37, 1984, Seiten 4147-4150, GB; J. CERVELLO et al.: "Nitrophenyl ethers as possible photoaffinitY labels. Te nucleophilic aromatic photosubstitution revisited"**
**PATENT ABSTRACTS OF JAPAN, Band 6, Nr. 110 (C-109)[988], 22. Juni 1982; & JP - A - 57 38755 (MITSUBISHI KASEI KOGYO) 03.03.1982**

(73) Patentinhaber: **HOECHST AKTIENGESELLSCHAFT, Postfach 80 03 20, D-6230 Frankfurt am Main 80 (DE)**

(72) Erfinder: **Koller, Wolfgang, Dr., Im Brühl 14, D-6231 Sulzbach (DE)**
Erfinder: **Papenfuhs, Theodor, Dr., Heinrich-Bleicher-Strasse 40, D-6000 Frankfurt am Main 50 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der vorliegenden Anmeldung ist ein Verfahren zur Herstellung von reinen 2-Ethylamino-4-nitro-1-alkoxybenzolen durch selektive Mono-N-Alkylierung von 1-Alkoxy-2-amino-4-nitrobenzolen mit Diethylsulfat.

Es ist bekannt, 1-Alkoxy-2-amino-4-nitrobenzole durch partielle Reduktion von 2,4-Dinitroalkoxybenzolen (Beilstein XIII, Seite 389) oder durch Nitrierung von o-Acetaminophenolethern und nachfolgende Entacylierung (Beilstein E II, 13, Seite 193) herzustellen. Die N-Alkylierung der so hergestellten 1-Alkoxy-2-amino-4-nitrobenzole führt jedoch, wie eigene Untersuchungen zeigten, nicht zu einheitlichen Produkten, sondern zu Produktgemischen aus jeweils nichtalkylierten primären Aminen, N-monoalkylierten sekundären Aminen und N,N-dialkylierten tertiären Aminen. Dies deckt sich mit Angaben in der Literatur, wonach die N-Alkylierung primärer aromatischer Amine normalerweise zu einer Mischung aus den drei genannten Verbindungstypen führt (R.J. Lindsay in «Comprehensive Organic Chemistry» Pergamon Press, Oxford, 1979, Band 2, Seite 161).

Dies hatte zwangsläufig zur Folge, dass die zur Herstellung wertvoller blauer Dispersionsazofarbstoffe erforderliche Kupplungskomponente in Form der Acylverbindung der Formel

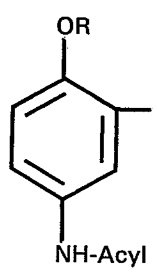

worin R die weiter unten genannte Bedeutung hat und $R_1$ eine niedere Alkylgruppe darstellt (erhältlich durch Reduktion und anschliessende Acylierung der 2-Alkylamino-4-nitro-1-alkoxyphenole), ebenfalls nicht als einheitliche Verbindung, sondern nur in Form eines entsprechenden Amingemisches eingesetzt werden konnte, was zu schlechten Farbstoffausbeuten und abgetrübten Farbstoffmischungen führte.

Wollte man keine Farbstoffgemische, sondern einheitliche Dispersionsazofarbstoffe erhalten, so musste man bisher zur Erzielung der angestrebten Monoalkylierung mit Schutzgruppen arbeiten. So kann beispielsweise die am aromatischen Kern sitzende Aminogruppe der 1-Alkoxy-2-amino-4-nitrobenzole mit Ameisensäure in die N-Formylgruppe überführt werden (EP 0 019 158). Die so erhaltene N-Formylverbindung kann anschliessend mit Dialkylsulfat alkyliert werden und die N-Formylgruppe kann dann mit Alkalilauge wieder abgespalten werden.

Es wurde nun überraschenderweise gefunden, dass man reine 2-Ethylamino-4-nitro-1-alkoxybenzole der Formel (1)

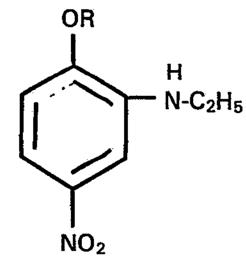

(1)

in welcher R eine Alkyl$_{C_1-C_4}$ oder Alkoxy$_{C_1-C_4}$alkyl$_{C_1-C_4}$gruppe bedeutet herstellen kann, indem man 2-Amino-4-nitro-1-alkoxybenzole der Formel (2)

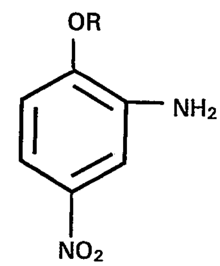

(2)

in welcher R die vorstehend genannte Bedeutung hat (ohne vorausgehende Einführung und spätere Abspaltung von Schutzgruppen) in unpolaren organischen Lösungsmitteln in Abwesenheit von Wasser mit einem geringen Überschuss an Diethylsulfat selektiv N-monoalkyliert.

Als geeignete unpolare organische Lösungsmittel kommen beispielsweise in Frage Toluol, Chlortoluole, Chlorbenzole, Xylole, Schwerbenzin, Naphtha (= technisches Gemisch aus Äthylbenzol und Xylolen) sowie Mischungen aus den vorstehend genannten Lösungsmitteln.

Demgegenüber entstehen beim Alkylieren in polaren Lösungsmitteln, wie beispielsweise Methanol oder Wasser, beträchtliche Mengen von nichtalkyliertem und N-dialkyliertem Amin.

Das erfindungsgemässe Verfahren wird im einzelnen wie folgt durchgeführt:

Zu einer Mischung aus 1 Mol 2-Amino-4-nitro-1-alkoxybenzol der vorstehend genannten Formel (2) in 300 bis 600 ml, vorzugsweise 400 ml eines unpolaren organischen Lösungsmittels, wie beispielsweise Schwerbenzin, Naphtha oder Toluol, lässt man 1,1 bis 1,5 Mol Diethylsulfat zulaufen und rührt die Mischung 1 bis 24 Stunden, vorzugsweise 5 Stunden bei etwa 40°C bis etwa 120°C, vorzugsweise bei 80°C. Dann wird mit 5-15%igem Ammoniakwasser neutralisiert, das eingesetzte organische Lösungsmittel durch azeotrope Destillation entfernt und der wässrige Rückstand mit konzentrierter Salzsäure auf pH 0,4 gestellt. Die angefallene Mischung wird mit Filtrierhilfsmittel, z.B. Kieselgur, und geringen Mengen Zinkstaub versetzt, 20-60 Minuten, vorzugsweise 30 Minuten unter Rückfluss gekocht und dann heiss geklärt. Schliesslich wird das Filtrat mit konzentrierter Natronlauge auf pH 2 gestellt und der Niederschlag abgesaugt und im Vakuum getrocknet.

Nach dem erfindungsgemässen Verfahren werden die 2-Ethylamino-4-nitro-1-alkoxybenzole der genannten Formel (1) rein und in hoher Ausbeute erhalten. Die Ausbeute beträgt 85-90% der Theorie, bezogen auf eingesetztes 2-Amino-4-nitro-1-alkoxybenzol der Formel (2). Die so erhaltenen 2-Ethylamino-4-nitro-1-alkoxybenzole lassen sich in bekannter Weise durch katalytische Hydrierung und nachfolgende Acylierung in hohen Ausbeuten in die technisch wertvollen, einheitlichen, entsprechenden 2-Ethylamino-4-acylamino-1-alkoxybenzole, die als Kupplungskomponenten zur Herstellung blauer Dispersionsazofarbstoffe dienen, überführen (Belgische Patentschrift 634 032). Durch den Erhalt der Verbindungen der Formel (1) in reiner Form ist es über die daraus herstellbaren 2-Ethylamino-4-acylamino-1-alkoxybenzole möglich, auch die blauen Dispersionsazofarbstoffe in einheitlicher Form und in guten Ausbeuten sowie besseren coloristischen Eigenschaften herzustellen. Im übrigen können durch die erfindungsgemässe Gewinnung der Verbindungen der Formel (1) in reiner Form auch die sonst beim Anfallen der Aminmischungen in der Produktion auftretenden Schwierigkeiten vermieden werden.

*Beispiel 1*

(2-Ethylamino-4-nitro-β-methoxyethoxy-benzol)

Zu einer Mischung aus 579 g Schwerbenzin (Fraktion 120-130°C) und 212 g 2-Amino-4-nitro-β-methoxy-ethoxybenzol (1 Mol) werden 200 g Diethylsulfat (1,3 Mol) zugetropft. Die Mischung wird 5 Stunden bei 80°C gerührt und dann mit 169 g 14%igem Ammoniakwasser neutralisiert. Das Schwerbenzin wird durch azeotrope Destillation entfernt und der wässrige Rückstand mit 165 g konzentrierter Salzsäure auf pH 0,4 gestellt. Die Mischung wird mit 10 g Kieselgur und 0,5 g Zinkstaub versetzt. Dann wird 30 Minuten unter Rückfluss gekocht und schliesslich heiss geklärt. Das Filtrat wird mit 120 ml konzentrierter Natronlauge auf pH 2 gestellt. Der Niederschlag wird abgesaugt und im Vakuum getrocknet.

Gewonnen werden 230,4 g an 2-Ethylamino-4-nitro-β-methoxy-ethoxybenzol, was einer Ausbeute von 88% der Theorie entspricht.

Schmelzpunkt: 83-87°C

*Gaschromatographische Analyse:*

| | |
|---|---|
| 2-Amino-4-nitro-β-methoxyethoxybenzol | 5% |
| 2-Ethylamino-4-nitro-β-methoxyethoxy-benzol | 92% |
| 2-Diethylamino-4-nitro-β-methoxyethoxy-benzol | 2% |

*Beispiel 2*

(2-Ethylamino-4-nitro-β-methoxyethoxy-benzol)

Zu einer Mischung aus 577 g Toluol und 212 g 2-Amino-4-nitro-β-methoxy-ethoxybenzol (1 Mol) werden 185 g Diethylsulfat (1,2 Mol) zugetropft. Die Mischung wird 5 Stunden bei 80°C gerührt und dann mit 169 g 14%igem Ammoniakwasser neutralisiert. Das Toluol wird durch azeotrope Destillation entfernt und der wässrige Rückstand mit 165 g konzentrierter Salzsäure auf pH 0,4 gestellt. Die Mischung wird mit 10 g Kieselgur und 0,5 g Zinkstaub versetzt. Dann wird 30 Minuten unter Rückfluss gekocht und schliesslich heiss geklärt. Das Filtrat wird mit 120 ml konzentrierter Natronlauge auf pH 2 gestellt. Der Niederschlag wird abgesaugt und im Vakuum getrocknet.

Gewonnen werden 215 g 2-Ethylamino-4-nitro-β-methoxyethoxybenzol, was einer Ausbeute von 76% der Theorie entspricht.

Schmelzpunkt: 82-85°C

*Gaschromatographische Analyse:*

| | |
|---|---|
| 2-Amino-4-nitro-β-methoxyethoxybenzol | 9% |
| 2-Ethylamino-4-nitro-β-methoxyethoxy-benzol | 85% |
| 2-Diethylamino-4-nitro-β-methoxyethoxy-benzol | 4% |

**Patentansprüche**

1. Verfahren zur Herstellung von reinen 2-Ethylamino-4-nitro-1-alkoxybenzolen der Formel (1)

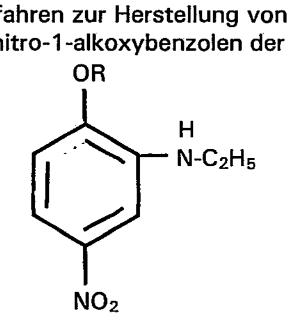

(1)

in welcher R eine Alkyl$_{C_1-C_4}$ oder Alkoxy$_{C_1-C_4}$alkyl$_{C_1-C_4}$gruppe bedeutet, dadurch gekennzeichnet, dass man 2-Amino-4-nitro-1-alkoxybenzole der Formel (2)

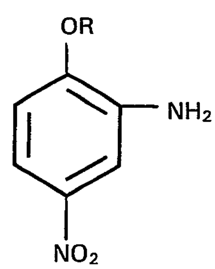

(2)

in welcher R die vorstehend genannte Bedeutung hat, in unpolaren organischen Lösungsmitteln in Abwesenheit von Wasser mit einem geringen Überschuss an Diethylsulfat bei Temperaturen von etwa 40 bis etwa 120°C N-monoalkyliert.

2. Verfahren nach Anspruch 1, dadurch gekennzeichnet, dass man in Toluol, Chlortoluolen, Chlorbenzolen, Xylolen, Schwerbenzin, Naphtha oder in Mischungen davon alkyliert.

**Claims**

1. A process for the preparation of pure 2-ethyl-

amino-4-nitro-1-alkoxybenzenes of the formula (1)

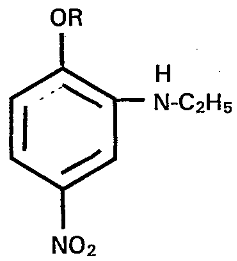

(1)

in which R denotes an alkyl($C_1$-$C_4$) or alkoxy($C_1$-$C_4$)-alkyl($C_1$-$C_4$) group, which comprises N-monoalkylation of 2-amino-4-nitro-1-alkoxybenzenes of the formula (2)

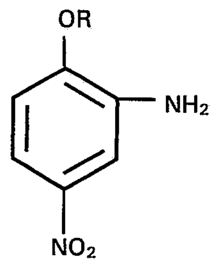

(2)

in which R has the abovementioned meaning, using a slight excess of diethyl sulfate in non-polar, organic solvents in the absence of water at temperatures of about 40 to about 120°C.

2. The process as claimed in claim 1, which comprises alkylation in toluene, chlorotoluenes, chlorobenzenes, xylenes, heavy gasoline, naphtha or mixtures of these.

**Revendications**

1. Procédé pour préparer des alcoxy-1 éthylamino-2 nitro-4 benzènes purs répondant à la formule (1)

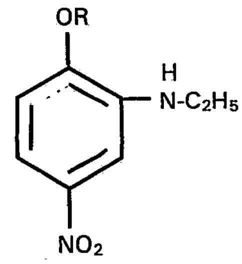

(1)

dans laquelle R représente un radical alkyle en $C_1$-$C_4$ ou un radical alcoxy-alkyle dont chacune des parties alcoxy et alkyle contient de 1 à 4 atomes de carbone, procédé caractérisé en ce qu'on monoalkyle à l'azote des alcoxy-1 amino-2 nitro-4 benzènes répondant à la formule (2)

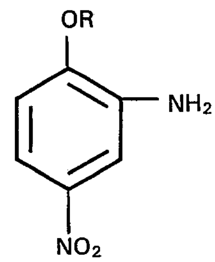

(2)

dans laquelle R a la signification indiquée ci-dessus, dans des solvants organiques non polaires, en l'absence d'eau, au moyen d'un léger excès de sulfate de diéthyle, à des températures d'environ 40 à environ 120°C.

2. Procédé selon la revendication 1, caractérisé en ce qu'on effectue l'alkylation dans du toluène, des chlorotoluènes, des chlorobenzènes, des xylènes, de l'essence lourde ou du naphtha, ou dans des mélanges de ces solvants.